# EUROPEAN PATENT SPECIFICATION

(11) **EP 3 397 244 B1**
(45) Date of publication and mention of the grant of the patent: **06.10.2021**
(21) Application number: 16881378.0
(22) Date of filing: 28.12.2016
(51) Int. Cl.: A61K 9/127, A61K 31/337, A61K 31/7068, A61K 47/18, A61K 47/24, A61P 35/00, C07D 305/14, C07H 19/06, C07H 19/073, A61K 45/06, A61K 9/00

(54) **TREATMENT OF BREAST CANCER USING A COMBINATION OF A CATIONIC LIPOSOMAL FORMULATION OF TAXANE, A NON-LIPOSOMAL FORMULATION OF TAXANE AND A FURTHER ACTIVE AGENT**
BEHANDLUNG VON BRUSTKREBS MIT EINER KOMBINATION AUS EINER KATIONISCHEN LIPOSOMALEN FORMULIERUNG VON TAXAN, EINER NICHT-LIPOSOMALEN FORMULIERUNG VON TAXAN UND EINEM WEITEREN WIRKSTOFF
TRAITEMENT DU CANCER DU SEIN AU MOYEN D'UNE ASSOCIATION D'UNE FORMULATION DE TAXANE LIPOSOMALE CATIONIQUE, D'UNE FORMULATION DE TAXANE NON LIPOSOMALE ET D'UN AUTRE PRINCIPE ACTIF

(30) Priority: 30.12.2015 US 201562272772 P
(43) Date of publication of application: 07.11.2018
(73) Proprietor: Syncore Biotechnology Co., Ltd., Yilan County 26944 (TW); Cancap Pharmaceutical Ltd., Richmond, British Columbia (CA)
(72) Inventor: LIN, Sih-Ting, Taipei City 110 (TW); TSENG, Hui-Yuan, Taipei City 110 (TW); TENG, Hsin-Wei, Taipei City 110 (TW)
(74) Representative: Weickmann & Weickmann PartmbB
(86) International application number: PCT/IB2016/058055
(87) International publication number: WO 2017/115301

(56) References cited:
- CA-A1- 2 922 029
- US-A1- 2009 047 337
- US-A1- 2009 186 078
- Anonymous: "A Trial Evaluating the Efficacy and Safety of EndoTAG -1 in Combination With Paclitaxel and Gemcitabine Compared With Paclitaxel and Gemcitabine as First-line Therapy in Patients With Visceral Metastatic Triple-negative Breast Cancer", ClinicalTrials.gov, NCT03002103, 23 December 2016 (2016-12-23), XP055557939, Retrieved from the Internet: URL:https://clinicaltrials.gov/ct2/show/NC T03002103 [retrieved on 2019-02-18]
- MICHAIL IGNATIADIS ET AL: "Feasibility Study of EndoTAG-1, a Tumor Endothelial Targeting Agent, in Combination with Paclitaxel followed by FEC as Induction Therapy in HER2-Negative Breast Cancer", PLOS ONE, vol. 11, no. 7, 25 July 2016 (2016-07-25), pages 1-11, XP055398938, DOI: 10.1371/journal.pone.0154009
- Anonymous: "Trial of Neoadjuvant EndoTAG-1 in Combination With Paclitaxel in HER2-negative Breast Cancer (EndoTAG-1)", ClinicalTrials.gov, NCT01537536, 23 February 2012 (2012-02-23), XP055557944, Retrieved from the Internet: URL:https://clinicaltrials.gov/ct2/show/NC T01537536 [retrieved on 2019-02-18]
- A. AWADA ET AL: "A randomized controlled phase II trial of a novel composition of paclitaxel embedded into neutral and cationic lipids targeting tumor endothelial cells in advanced triple-negative breast cancer (TNBC)", ANNALS OF ONCOLOGY., vol. 25, no. 4, 25 March 2014 (2014-03-25) , pages 824-831, XP055398942, NL ISSN: 0923-7534, DOI: 10.1093/annonc/mdu025
- "A trial evaluating the efficacy and safety of EndoTag®-1 in combination with paclitaxel and gemcitabine compared with paclitaxel and gemcitabine as fust-line therapy in patients with visceral metatstatic triple-negative breast cancer", ClinicalTrial, 22 December 2016 (2016-12-22), XP055557939, Retrieved from the Internet: URL:https://clinicaltrials.gov/ct2/show/NC T03002103
- IGNATIADIS et al.: "Feasibility study of EndoTAG-1, a tumor endothelial targeting agent, in combination with paclitaxel followed by FEC as induction therapy in HER2-negative breast cancer", PLOS ONE, vol. 10, 25 July 2016 (2016-07-25), pages 1-11, XP055398938,
- "Trial of neoadjuvant EndoTAG-1 in combination with paclitaxel in HER2-negative breast cancer (EndoTAG-1", ClinicalTrial, 22 February 2012 (2012-02-22), XP055557944, Retrieved from the Internet: URL:https://clinicaltrials.gov/ct2/show/NC T01537536?term=NCT01537536&rank=1
- AWADA et al.: "A randomized controlled phase II trial of a novel composition of paclitaxel embedded into neutral and cationic lipids targeting tumor endothelial cells in advanced triple-negative breast cancer (TNBC)", Annals of Oncology, vol. 25, no. 4, 2014, pages 824-831, XP055398942,
- LÖHR et al.: "Cationic liposomal paclitaxel plus gemcitabine or gemcitabine alone in patients with advanced pancreatic cancer: A randomized controlled phase II trial", Annals of Oncology, vol. 23, 2012, pages 1214-1222, XP055398947,
- EICHHORN et al.: "Vascular targeting by EndoTAG?-1 enhances therapeutic efficacy of conventional chemotherapy in lung and pancreatic cancer", International Journal of Cancer, vol. 126, 2010, pages 1235-1245, XP055398949,
- DEL MASTRO et al.: "Randomised phase 3 open-label trial of fust-line treatment with gemcitabine in association with docetaxel or paclitaxel in women with metastatic breast cancer: A comparison of different schedules and treatments", BMC Cancer, vol. 13, 2013, pages 1-10, XP021146618,
- MAVROUDIS et al.: "Front-line chemotherapy with docetaxel and gemcitabine adminstered every two weeks in patients with metastatic breast cancer: A multicenter phase II study", Oncology, vol. 67, 2004, page 250256, XP009511391,
- KONTOPODIS et al.: "Docetaxel, gemcitabine and bevacizumab as salvage chemotherapy for HER-2 negative metastatic breast cancer", Cancer Chemother . Pharmacol., vol. 75, no. 1, 2015, pages 153-160, XP035416477,
- ANAMPA et al.: "Progress in adjuvant chemotherapy for breast cancer: An overview", BMC Medicine, vol. 13, 2015, pages 1-13, XP021227369,

## Description

### TECHNICAL FIELD

The present disclosure provides a combination for use in methods for treating breast cancer.

### BACKGROUND

Breast cancer remains the most frequently diagnosed cancer and one of the leading causes of cancer death among females worldwide, despite screening and improvements in therapy. The treatment of breast cancer depends on various factors including stage of the cancer and age of the patient. Breast cancer is usually treated with surgery, such as lumpectomy or mastectomy followed by medication and/or radiation therapy. Medication includes hormone-blocking agents, chemotherapeutic agents, and monoclonal antibodies.

Breast cancer begins in different areas of the breast such as the ducts, the lobules, or the tissues in between. Thus, the different types of breast cancer depend upon where the cancer cells originate and can be characterized as non-invasive, invasive, recurrent, and metastatic. Examples of the different types of breast cancer include ductal carcinoma in situ (DCIS), invasive ductal carcinoma (IDC), inflammatory breast cancer, and triple negative breast cancer. DCIS is a non-invasive cancer in which abnormal cells are found in the lining of the breast milk duct. IDC, sometimes called infiltrating ductal carcinoma, is the most common type of breast cancer. Inflammatory breast cancer is an aggressive and fast growing breast cancer in which cancer cells infiltrate the skin and lymph vessels of the breast.

Breast cancer includes several different carcinomas, which are classified for therapeutic reasons according to their estrogen receptor (ER), progesterone receptor (PrR), and human epidermal growth factor receptor 2 (HER2) status. Targeted therapies against ER, PrR, and HER2 are available. However, these treatment options are absent for patients with tumors lacking these receptor targets, which are referred to as triple-negative breast cancer (TNBC). TNBC is an aggressive subtype with limited treatment options and very poor prognosis following progression after standard anthracycline or taxane regimens. About 15% of all breast cancer cases are negative for ER, PgR and HER-2. In these cases prognosis is very poor with an 80% relapse rate and a median survival of only 6 months.

According to international guidelines, conventional systemic chemotherapy based on the existing cytotoxic agents remains the only treatment option for patients with advanced TNBC (Cardoso F, Costa A, Norton L, et al: 1st International consensus guidelines for advanced breast cancer (ABC 1). Breast. 21:242-252, 2012). However, there is no robust, prospective, randomized data supporting the use of specific chemotherapeutic regimens. As a result of lacking evidence, there are no specific recommendations to manage these patients in clinical practice. The European Society for Medical Oncology guidelines refer to taxane-based regimen as first-line therapy in patients progressing after adjuvant anthracycline-based therapy being the only standard of care with level-1 evidence.

Taxanes are chemotherapeutic agents that have been used to treat breast cancer. However, taxanes induce adverse side effects. Moreover, with time and repeated use, it has been reported that cancer cells develop resistance to taxanes, similar to other chemotherapeutic agents, thereby providing no further benefit to the patient. The conventional strategy to deal with the issue of drug resistance is to increase the dose of taxane to the maximal tolerated dose (MTD) in an attempt to eradicate all tumor cells as quickly and completely as possible. However, this strategy causes severe adverse effects and cannot be used under extended period of time. Accordingly, the treatment consists of cycles of short treatment period of usually one day a week at MTD, followed by a treatment free interval of several weeks to allow the patient to recover. It has been reported that during the treatment free interval, tumor growth can restart. The common solution has been to start the treatment with a second drug. However, the tumors often only respond for a certain amount of time leading to only a temporary regression of the tumor. Consequently, the tumors become also resistant to the second drug. Continuing with this strategy only leads to development of multi-drug resistant tumors that are finally refractory to all available anti-cancer drugs.

Combination therapy, e.g., combining one or more chemotherapeutic agents, rather than single-agent therapy is considered for patients with aggressive or visceral metastatic disease, particularly with regard to their bad prognosis of survival. For example, in 2004, gemcitabine (1250 mg/m² on days 1 and 8 of a 3-week cycle), in combination with paclitaxel (175 mg/m² on day 1 of a 3-week cycle), was approved for first-line treatment of metastatic breast cancer. However, paclitaxel with extremely low solubility in water is formulated and administered in a vehicle containing Cremophor EL (a polyethoxylated castor oil), which induces various side effects as paclitaxel is poorly soluble in water.

Furthermore, adverse effect may be more common in combination therapy than single-agent therapy. For example, in a phase III study comparing gemcitabine (1250 mg/m² on days 1 and 8)/paclitaxel (175 mg/m² on day 1) with triweekly paclitaxel (175 mg/m²) alone as the first-line therapy in metastatic breast cancer patients, a higher incidence of Grade 3/4 neutropenia (48 vs. 11%) and febrile neutropenia (5 vs. 1.2%) was reported in patients in the combination arm, also a greater frequency of non-hematologic toxicity such as asthenia (7 vs. 2%), alteration of hepatic function (7 vs. 2%) and peripheral neuropathy (6 vs. 4%) (Albain KS, Nag SM, Calderillo-Ruiz G, Jordaan JP, Llombart AC, Pluzanska A et al. Gemcitabine plus Paclitaxel versus Paclitaxel monotherapy in patients with metastatic breast cancer and prior anthracycline treatment. J Clin Oncol. 2008;26:3950-7.).

Further, the webpage clinicaltrials.gov summarizes the set-up for a prospective, single-center, open-label II clinical trial investigating the activity of Endo TAG-1 and paclitaxel combination therapy in patients with HER2-negative breast cancer as candidate for new adjuvant chemotherapy. No results are provided. No combination with gemcitabine as further active agent is disclosed.

US 2009/0186078 A1 relates to the use of a cationic liposomal preparation comprising at least one cationic lipid and an antimitotic agent for the treatment of triple receptor negative breast cancer, wherein the antimitotic agent might be taxane such as paclitaxel or a derivative thereof. No combination comprising a cationic liposomal formulation with paclitaxel, non-liposomal paclitaxel and gemcitabine is disclosed.

US 2009/0047337 A1 relates to EndoTAG-1 in combination with gemcitabine for the treatment of cancer. No combination comprising a cationic liposomal formulation with paclitaxel, non-liposomal paclitaxel and gemcitabine is disclosed.

Awada et al, 2014, Annals of Oncology 25:824-831 relates to a random controlled phase II trial on patients with advanced triple-negative breast cancer. The patients were administered with EndoTAG-1 and paclitaxel. No combination comprising a cationic liposomal formulation with paclitaxel, a non-liposomal formulation with paclitaxel and gemcitabine is disclosed.

Therefore, for taxane-based therapy, there is a need to develop a method and/or dosage regimen to deliver taxane with enhanced effect and reduced side effects.

### SUMMARY

Methods of treating breast cancer by administering taxane to a subject in need thereof in a therapeutically effective amount without severe adverse effects such as those caused by administering high initial treatment doses of taxane in Cremophor EL formulations are described herein.

The methods described herein comprise administering to a subject in need thereof a therapeutically effective amount of a cationic liposomal formulation comprising one or more cationic lipids and a taxane. The method can further comprise administering one or more non-liposomal formulations including one or more further active agents.

The scope of the invention is defined by the appended claims.

The present invention provides a combination comprising (a) a cationic liposomal formulation comprising one or more cationic lipids and a therapeutically effective amount of taxane; and (b) a non-liposomal formulation comprising a therapeutically effective amount of taxane; and (c) a therapeutically effective amount of a further active agent for use in a method of treating breast cancer wherein the taxane is paclitaxel and the further active agent is gemcitabine.

In some embodiments, the combination for use in a method of present disclosure relates to a dosage regimen comprising at least one cycle, wherein the cycle is a period of 28 days, and wherein for each cycle the cationic liposomal formulation is administered on days 1, 8 and 15 of the cycle at a dose of about 1 to 60 mg/m² taxane; the non-liposomal is administered on days 1, 8 and 15 of the cycle at a dose of about 5 to 100 mg/m² taxane; and the further active agent gemcitabine is administered on days 1 and 8 of the cycle at a dose of about 100 to 1250 mg/m².

In embodiments, the combination for use in a method of treating breast cancer includes administering to a subject, a cationic liposomal formulation including about 11 mg/m² to about 22 mg/m² of taxane.

In embodiments, the combination for use in a method of treating breast cancer includes administering to a subject, non-liposomal formulation including about 70 mg/m² to about 90 mg/m² taxane.

In embodiments, the combination for use in a method of treating breast cancer includes administering to a subject, a further active agent gemcitabine at about 800 mg/m² to about 1250 mg/m².

In embodiments, the combination for use in a method of treating breast cancer comprises administering the cationic liposomal formulation to the subject prior to the non-liposomal formulation, and the method comprises administering the non-liposomal formulation to the subject prior to gemcitabine.

In some embodiments, the hormone receptor status of the subject is negative for both estrogen receptor and progesterone receptor. In other embodiments, the breast cancer is HER2 positive (HER2+). In particular embodiments, the breast cancer is HER2 negative (HER2-).

### BRIEF DESCRIPTION OF THE DRAWINGS

FIG. 1 shows activity of drug combinations in a TNBC tumor model.
FIG. 2 is a schematic illustration of a clinical trial design.

### DETAILED DESCRIPTION OF PREFERRED EMBODIMENTS

As used herein, the term "therapeutically effective amount" is an amount of an active agent, that is sufficient to achieve the desired therapeutic result in the treated subject. The result can be reduction, amelioration, palliation, lessening, delaying, and/or alleviation of one or more of the signs, symptoms, or causes of a disease. In some embodiments, a therapeutically effective amount comprises an amount sufficient to cause a tumor to shrink or to decrease growth rate. In some embodiments, a therapeutically effective amount is an amount sufficient to prevent or delay tumor recurrence. In some embodiments, a therapeutically effective amount is an amount sufficient to inhibit, retard, slow to some extent and may stop cancer cell infiltration into peripheral organs; inhibit (i.e., slow to some extent and may stop) tumor metastasis; inhibit tumor growth; prevent or delay occurrence and/or recurrence of tumor. A therapeutically effective amount can be administered in one or more administrations.

As used herein, the term "subject" refers to a mammal, for example a human or an animal. In embodiments, the subject is in need of a treatment (in need thereof) and the subject is a human cancer patient. In particular embodiments, the subject in need of a treatment (in need thereof) is a subject having breast cancer. In embodiments, the subject is a human patient diagnosed with or suffering from breast cancer, for example DCIS, IDS, inflammatory breast cancer, and triple negative breast cancer. In particular embodiments, the subject is a human patient that has been diagnosed with triple negative breast cancer. The human patient may be pre- or post-menopausal. The cancer to be treated can be in different clinical stages according to size, distribution, and degree of metastasis formation.

As used herein a "dosage regimen" refers to a protocol used to administer a liposomal formulation or non-liposomal formulation to a subject. A dosage regimen comprises a dose and dosing interval. A dosage regimen further comprises a dosing duration. As used herein "dose" refers to an amount of an active agent given in a single administration. The interval between doses can be a desired amount of time and is referred to as the "dosing interval". As used herein "dosing duration" refers to the period of time over which a dose is administered.

The unit "mg/m²" refers to an amount of an active agent per human body surface area (m²). The dose calculation refers only to the mass of the active agent, not the lipid portion.

The term "combination therapy" as used herein includes simultaneous administration of at least two active agents to a subject or their sequential administration within a time period during which the first administered therapeutic agent is still present in the subject when the second administered therapeutic agent is administered.

The term "liposome" refers to a microscopic spherical membrane-enclosed vesicle (about 50-2000 nm diameter). The term "liposome" encompasses any compartment enclosed by a lipid bilayer. Liposomes are also referred to as lipid vesicles. In order to form a liposome the lipid molecules comprise elongated non polar (hydrophobic) portions and polar (hydrophilic) portions. The hydrophobic and hydrophilic portions of the molecule are preferably positioned at the two ends of an elongated molecular structure. When such lipids are dispersed in water they spontaneously form bilayer membranes referred to as lamellae. The lamellae are composed of two mono layer sheets of lipid molecules with their non-polar (hydrophobic) surfaces facing each other and their polar (hydrophilic) surfaces facing the aqueous medium. The membranes formed by the lipids enclose a portion of the aqueous phase in a manner similar to that of a cell membrane enclosing the contents of a cell.

Thus, the bilayer of a liposome has similarities to a cell membrane without the protein components present in a cell membrane. As used herein, the term liposome includes multilamellar liposomes, which generally have a diameter in the range of about 1 to 10 micrometers and having anywhere from two to hundreds of concentric lipid bilayers alternating with layers of an aqueous phase, and also includes unilamellar vesicles which are a single lipid layer and have a diameter in the range of about 20 to about 400 nanometers (nm), about 50 to about 300 nm, about 300 to about 400 nm, or about 100 to about 200 nm, which vesicles can be produced by subjecting multilamellar liposomes to ultrasound, by extrusion under pressure through membranes having pores of defined size, or by high pressure homogenization. The liposomes can be unilamellar vesicles, which have a single lipid bilayer, and a diameter in the range of about 25-400 nm.

The cationic liposomal formulation provided herein includes one or more cationic lipids, a taxane, and optionally a neutral and/or anionic lipid. As used herein, the terms "liposome", "liposomal preparation", and "liposomal formulation" are used synonymously throughout the present application.

The amount of cationic lipids in the cationic liposomal formulation is from about 30 mole% to about 99.9 mole%. The amount of taxane in the cationic liposomal formulation is at least about 0.1 mole%. The amount of neutral and/or anionic lipid is from about 30 mole % to about 70 mole %.

In some embodiments, the amount of cationic lipids in the cationic liposomal formulation includes between about 40 mole% and about 95 mole%, about 50 mole% and about 90 mole%, about 60 mole% and about 85 mole%, about 65 mole% and about 75 mole%, or about 70 mole%.

In other embodiments, the cationic liposomal formulation includes a taxane in an amount of between about 0.5 mole% and about 10 mole%, about 1.0 mole% and about 8 mole%, about 2 mole% and about 6 mole%, about 2.5 mole% and about 5 mole%, or about 2.5 mole% and about 3.0 mole%.

Optionally, the cationic liposomal formulation includes neutral and/or anionic lipids, in an amount of between about 30 mole% and about 70 mole%, about 40 mole% and about 60 mole%, or about 45 mole% and about 55 mole%.

In embodiments, the liposomal formulation has a zeta potential in the range of about 0 mV to about 100 mV or in the range of about 20 mV to about 100 mV, in about 0.05 mM KCl solution at about pH 7.5.

As used herein, the term "zeta potential" refers to a measured electrical potential of a particle, such as a liposome, measured with an instrument, such as a Zetasizer 3000 using Laser Doppler micro-electrophoresis under the conditions specified. The zeta potential describes the potential at the boundary between bulk solution and the region of hydrodynamic shear or diffuse layer. The term is synonymous with "electrokinetic potential" because it is the potential of the particles which acts outwardly and is responsible for the particle's electrokinetic behavior.

The one or more cationic lipids in the cationic liposomal formulation are selected from the group consisting of N-[1-(2,3-dioleoyloxy)propyl]-N,N,N-trimethyl ammonium salts, such as N-[1-(2,3-dioleoyloxy)propyl]-N,N,N-trimethyl ammonium salt (DOTAP); dimethyldioctadecyl ammonium bromide (DDAB); 1,2-diacyloxy-3-trimethylammonium propanes, including for example, dioleoyl, dimyristoyl, dilauroyl, dipalmitoyl, and distearoyl, and including those with two different acyl chain linked to the glycerol backbone); N-[1-(2,3-dioloyloxy)propyl]-N,N-dimethyl amine (DODAP); 1,2-diacyloxy-3-dimethylammonium propanes, including for example, dioleoyl, dimyristoyl, dilauroyl, dipalmitoyl, and distearoyl, and including those with two different acyl chain linked to the glycerol backbone; N-[1-(2,3-dioleyloxy)propyl]-N,N,N-trimethylammonium chloride (DOTMA); 1,2-dialkyloxy-3-dimethylammonium propanes, including for example dioleyl, dimyristyl, dilauryl, dipalmityl, and distearyl and including those with two different alkyl chain linked to the glycerol backbone; dioctadecylamidoglycylspermine (DOGS); 3β-[N--(N',N'-dimethylamino-ethane)carbamoyl]cholesterol (DC-Chol); 2,3-dioleoyloxy-N-(2-(sperminecarboxamido)-ethyl)-N,N-dimethyl-1-propanam-inium trifluoro-acetate (DOSPA); β-alanyl cholesterol; cetyl trimethyl ammonium bromide (CTAB); diC14-amidine; N-tert-butyl-N'-tetradecyl-3-tetradecylamino-propionamidine; 14Dea2; N-(alpha-trimethylammonioacetyl)didodecyl-D-glutamate chloride (TMAG); O,O'-ditetradecanoyl-N-(trimethylammonio-acetyl)diethanolamine chloride; 1,3-dioleoyloxy-2-(6-carboxy-spermyl)-propylamide (DOSPER); N,N,N',N'-tetramethyl-N,N'-bis(2-hydroxylethyl)-2,3-dioleoyloxy-1,4-butan-ediammonium iodide; 1-[2-(acyloxy)ethyl]-alkyl(alkenyl)-3-(2-hydroxyethyl)-imidazolinium chloride derivatives, such as 1-[2-(9(Z)-octadecenoyloxy)ethyl]-2-(8(Z)-heptadecenyl-3-(2-hydroxyethyl)-imidazolinium chloride (DOTIM) and 1-[2-(hexadecanoyloxy)ethyl]-2-pentadecyl-3-(2-hydroxyethyl)imidazolinium chloride (DPTIM); 2,3-dialkyloxypropyl quaternary ammonium compound derivatives, containing a hydroxyalkyl moiety on the quaternary amine, for example, 1,2-dioleoyl-3-dimethyl-hydroxyethyl ammonium bromide (DORI), 1,2-dioleyloxypropyl-3-dimethyl-hydroxyethyl ammonium bromide (DORIE), 1,2-dioleyloxypropyl-3-dimethyl-hydroxypropyl ammonium bromide (DORIE-HP), 1,2-dioleyloxypropyl-3-dimethyl-hydroxybutyl ammonium bromide (DORIE-HB), 1,2-dioleyloxypropyl-3-dimethyl-hydroxypentyl ammonium bromide (DORIE-Hpe), 1,2-dimyristyloxypropyl-3-dimethyl-hydroxylethyl ammonium bromide (DMRIE), 1,2-dipalmityloxypropyl-3-dimethyl-hydroxyethyl ammonium bromide (DPRIE), and 1,2-disteryloxypropyl-3-dimethyl-hydroxyethyl ammonium bromide (DSRIE); cationic esters of acyl carnitines; and cationic triesters of phospahtidylcholine, for example, 1,2-diacyl-sn-glycerol-3-ethylphosphocholines, in which the hydrocarbon chains are saturated or unsaturated and branched or unbranched with a chain length from C₁₂ to C₂₄, and the two acyl chains may or may not be identical.

Optionally, the liposomal preparation comprises one or more neutral and/or anionic lipids. The neutral and anionic lipids are selected from sterols or lipids such as cholesterol, phospholipids, lysolipids, lysophospholipids, sphingolipids, or pegylated lipids with a neutral or negative net change. In particular embodiments, the neutral and anionic lipids include: phosphatidylserine; phosphatidylglycerol; phosphatidylinositol; fatty acids; sterols containing a carboxylic acid group for example, cholesterol; 1,2-diacyl-sn-glycero-3-phosphoethanolamines, including DOPE; 1,2-diacyl-glycero-3-phosphocholines; and sphingomyelin. The fatty acids linked to the glycerol backbone have various length and number of double bonds. Phospholipids can have two different fatty acids. In embodiments, the neutral and/or anionic lipids are in the liquid crystalline state at room temperature and they are miscible with the used cationic lipid, in the ratio as they are applied. The neutral and/or anionic lipids and the cationic lipids can form a uniform phase and no phase separation or domain formation occurs. In embodiments, the neutral lipid is DOPC (1,2-dioleoyl-sn-glycero-3-phosphocholine).

The liposomal and non-liposomal formulations include taxanes. As used herein, the term "taxane" herein refers to a class of antineoplastic agents having the function of binding microtubules which inhibit cell division and having a structure that includes the taxane ring structure and a stereospecific side chain that is required for cytostatic activity. The term taxane also includes a variety of known derivatives, such as hydrophilic derivatives and hydrophobic derivatives. Taxane derivatives include galactose and mannose derivatives described in International Patent Application No. WO 99/18113; piperazino and other derivatives described in WO 99/14209; taxane derivatives described in WO 99/09021, WO 98/22451, and U.S. Pat. No. 5,869,680; 6-thio derivatives described in WO 98/28288; sulfenamide derivatives described in U.S. Pat. No. 5,821,263; and taxol derivative described in U.S. Pat. No. 5,415,869. The taxane of the present invention is paclitaxel.

The term "paclitaxel" includes analogues, formulations, and derivatives such as, for example, docetaxel (Taxotere, a formulation of docetaxel), 10-desacetyl analogs of paclitaxel and 3'N-desbenzoyl-3'N-t-butoxycarbonyl analogs of paclitaxel. Paclitaxels can be readily prepared utilizing techniques known to those skilled in the art (see also WO 94/07882, WO 94/07881, WO 94/07880, WO 94/07876, WO 93/23555, WO 93/10076; U.S. Pat. Nos. 5,294,637; 5,283,253; 5,279,949; 5,274,137; 5,202,448; 5,200,534; 5,229,529; and EP 590,267), or obtained from a variety of commercial sources, including for example, Sigma Chemical Co., St. Louis, Mo. (T7402 from Taxus brevifolia; or T-1912 from Taxus yannanensis). Paclitaxel refers not only to the common chemically available form of paclitaxel (e.g. Taxol®), but also analogs (e.g., Taxotere, as noted above) and paclitaxel conjugates (e.g., paclitaxel-PEG, paclitaxel-dextran, or paclitaxel-xylose).

The term "derivative" refers to a compound derived from some other compound while maintaining its general structural features. Derivatives may be obtained for example by chemical functionalization or derivatization.

The term "liposomal paclitaxel" or "lipid complexed paclitaxel" refers to a liposomal preparation. A specific liposomal paclitaxel formulation is EndoTAG®-1. The manufacture of such a formulation is disclosed in WO 2004/002468, US7794747, US8075913, US8663606, and US9238021. EndoTAG®-1 is a liposomal preparation with a mole ratio of 50:47:3 mole % of DOTAP, DOPC and paclitaxel.

Due to the steric shape and the amphiphilic nature of the lipids, self-assembly leads to the formation of lipid bilayers (membranes), in which the hydrophobic alkyl chains are oriented toward each other and the polar head groups are oriented toward the aqueous phase. These membranes are organized as spherical vesicles, so-called liposomes. As a result of the presence of cationic (positively charged) lipid molecules in the bilayer membrane, the liposomes are cationic. EndoTAG®-1 is in the form of a lyophilized powder. It can be reconstituted with water for injection prior to application. The resulting solution consists of small liposomal vesicles with an intensity weighted average particle size of about less than 300 nm.

The cationic liposomal formulation described herein includes one or more cationic lipids, one or more neutral lipids, and a taxane. In embodiments, the cationic lipid is DOTAP; the neutral lipid is DOPC; and the taxane is paclitaxel. The mole ratio of cationic lipids, neutral lipids, and taxanes is in the range of about 40 to 60 cationic lipids, about 39 to 55 neutral lipids, and about 1 to 5 taxane. In particular embodiments, the cationic liposomal formulation includes DOTAP, DOPC, and paclitaxel in a mole ratio of about 50:47:3.

Other than administering taxanes in the cationic liposomal formulation and the non-liposomal formulation, the method described herein comprises administering a further active agent. The further active agent is not a paclitaxel or is not a taxane.

As used herein, the term "active agent" refers to an agent that is therapeutically effective. An active agent can be a cytotoxic or cytostatic substance such as an anti-tumor or an anti-endothelial cell active substance, a chemotherapeutic agent or an immunological active substance, a compound that reduces or eliminates hypersensitivity reactions, or a chemosensitizer or combinations thereof.

Examples of chemotherapeutic agents include those that are effective against breast cancer include folate antagonists, including methotrexate and pemetrexed; purine antagonists, including cladribine, clofarabine, fludarabine, 6-mercaptopurine, nelarabine, and pentostatin; pyrimidine antagonists, including capecitabine, cytarabine, 5-fluorouracil, gemcitabine, and hydroxyurea; biologic response modifiers, including interferon-alfa; bleomycin; DNA alkylating agents, including nitrosureas, carmustine, and lomustine; DNA cross-linking drugs and alkylating agents, including bendamustine, chlorambucil, cyclophosphamide, ifosfamide, mechlorethamine (nitrogen mustard), melphalan, dacarbazine, temozolomide, and procarbazine; asparaginase; antibiotics, including mitomycin; platinum complexes, including carboplatin, cisplatin, and oxaliplatin; proteosome inhibitors, including bortezomib; spindle poisons, such as the taxanes, such as docetaxel and paclitaxel, and the vincas, such as vinblastine, vincristine, and vinorelbine; topoisomerase inhibitors, such as anthracyclines (including daunorubicin, daunomycin, doxorubicin, epirubicin, valrubicin, and mitoxantrone), the camptothecins, (including irinotecan, and topotecan), and the podophyllotoxins (including etoposide, teniposide and mitoxantrone); tyrosine kinase inhibitors, (including erlotinib (Tarceva), gefitinib, imatinib, lapatinib, sorafenib, and sunitinib).

Combination of active agents that are effective for the treatment of breast cancer includes CMF, which is a combination of cyclophosphamide, methotrexate, and 5-fluorouracil; CAF (FAC), which is a combination of cyclophosphamide, doxorubicin, and 5-fluorouracil; AC, which is a combination of doxorubicin and cyclophosphamide; EC, which is a combination of epirubicin and cyclophosphamide; TAC, which is a combination of docetaxel, doxorubicin, and cyclophosphamide; AC→T, which is a combination of doxorubicin and cyclophosphamide followed by paclitaxel or docetaxel; Herceptin may be given with the paclitaxel or docetaxel for HER2 positive tumors; A→CMF, which is a combination of doxorubicin, followed by CMF; CEF (FEC), which is a combination of cyclophosphamide, epirubicin and 5-fluorouracil (this may be followed by docetaxel); TC, which is a combination of docetaxel and cyclophosphamide; and TCH, which is a combination of docetaxel, carboplatin, and Herceptin for HER2 positive tumors.

Other active agents can include compounds that reduce or eliminate hypersensitivity reactions. Examples of such compounds include steroids, antihistamines, H2 receptor antagonists, and combinations thereof in a sufficient amount to prevent fatal anaphylactic reactions. The compounds can include ranitidine, dexamethasone, diphenhydramine, famotidine, hydrocortisone, clemastine, cimetidine, prednisolone, chlorpheniramine, dimethindene maleate, and promethazine.

Other active agents can include chemosensitizers. As used herein, the term "chemosensitizer" refers to a substance or drug, which makes it easier for chemotherapy to affect, particularly kill cancer cells. Examples of chemosensitizers include cell cycle modulators, substances that revert drug resistance like verapamil, vasoactive substances like anti-hypertensive drugs, and substances that modify interactions of cationic liposomes with blood components like protamine. The further active agent of the present invention is gemcitabine.

The cationic liposomal formulation and/or the non-liposomal formulation can include one or more carriers. As used herein, the term "carrier" refers to a diluent, adjuvant, excipient, or vehicle which is suitable for administering a diagnostic or therapeutic agent. The term also refers to pharmaceutically acceptable carriers that contain, complexes or is otherwise associated with an agent to facilitate the transport of such an agent to its intended target site. Carriers include those known in the art, such as liposomes, polymers, lipid complexes, serum albumin, antibodies, cyclodextrins, dextrans, chelates, or other supramolecular assemblies.

The formulations, in particular the cationic liposomal formulation, disclosed herein can be provided in a dry, dehydrated, or lyophilized form. Prior to administration, the formulation can be hydrated in pharmaceutical grade water or saline or another suitable liquid, preferably comprising physiologically acceptable carriers such as a buffer.

The formulations disclosed herein can be provided in the form of kits. The kit can include a cationic liposomal formulation, a non-liposomal formulation and one or more further active agents. The one or more active further active agents can be a chemotherapeutic agent. The cationic and non-liposomal formulations in the kit include a taxane, such as paclitaxel, and the one or more further active agent includes gemcitabine. The kits can also include a container and/or reagents for preparing the formulations for administration. As an example, the cationic liposomal formulation can be in a dehydrated form that can be reconstituted by hydration.

The formulations provided herein can be used in a first-, second-, or third line treatment. Subjects who are treated may have undergone surgery for tumor resection (such as lumpectomy or mastectomy), radiotherapy, and/or chemotherapy. A common chemotherapy for breast cancer is anthracycline-based combination regimes. Typically this combination regimen involves an anthracycline drug, such as doxorubicine or epirubicine. As an example, the first line treatment can include a combination therapy of an anthracycline drug and a taxane drug, and the second line treatment can be the cationic liposomal formulation provided herein or the combination of the cationic liposomal formulation and the non-liposomal formulation provided herein.

As used herein, the term "combination" or "co-administration" refers to an administration schedule that is synchronous, serial, overlapping, alternating, parallel, or any other treatment schedule in which the various active agents or therapies are administered as part of a single treatment regimen, prescription or indication or in which the time periods during which the various agents or therapies that are administered otherwise partially or completely coincide.

The combination of liposomal formulation and non-liposomal formulation can be co-administered separately, simultaneously, or sequentially. The cationic liposomal formulation and the non-liposomal formulation can be administered at different time points on the same day or on different days. In embodiments the cationic liposomal formulation is administered prior to the non-liposomal formulation, for example, more than one hour, but not more than twelve hours prior to the administration of the non-liposomal formulation.

In embodiments, the liposomal formulation, the non-liposomal formulation, and the further active agent are administered sequentially. In particular embodiments, the liposomal formulation is administered first, the non-liposomal formulation is administered second, and the further active agent is administered third.

In embodiments, the administration rate of the cationic liposomal formulation is increased in a stepwise manner. The cationic liposomal formulation is administered at an initial rate, followed by one or more higher rates. The cationic liposomal formulation is administered at a rate of between about 0.3 to 0.7 mL/min during the first 15 minutes, at a rate of between about 0.8 to 1.2 mL/min during the second 15 minutes, and at a rate of between about 1.3 to 1.7 mL/min after 30 minutes. In particular embodiments, the cationic liposomal formulation is administered at a rate of about 0.5 mL/min during the first 15 minutes, at a rate of about 1.0 mL/min during the second 15 minutes, and at a rate of about 1.5 mL/min after 30 minutes.

The formulations can be administered to the subject at a therapeutically effective amount of at least once a week. In embodiments, the formulations are administered once or twice a week. In other embodiments, the formulations can be administered alternately once a week and twice a week during the treatment period.

Depending on the duration of the treatment and on the observed side effects, the administration of the formulations can also be omitted for at least one week or several weeks during the treatment period.

In embodiments, the methods described herein include administering the cationic liposomal formulation in a single dose of between about 1 mg/m² to about 60 mg/m².

As used herein, the unit "mg/m²", refers to mg of active agent, for example paclitaxel, per m² body surface (bs) of the subject.

As used herein, the unit mg/kg body weight of a subject or mg/kg refers to mg of active agent, for example paclitaxel, per kg body weight (bw) of a subject.

In embodiments, on an average, a human subject has a body surface of about 1.84 m². Thus, for an average person of 70 kg body weight and 172 cm height, values for single doses, monthly doses, etc. which are in mg/kg body weight (bw) may be converted for human applications to corresponding values of in mg/m² human body surface (bs) by multiplication with a species-specific factor according to known methods. Similarly, doses in mg/m² bs of a human subject can be converted to mg/kg bw of a human subject.

The use in the methods as disclosed herein comprises administering the cationic liposomal formulation in a dose of between about 1 mg/m² and about 50 mg/m², between about 25 mg/m² and about 50 mg/m², between about 10 mg/m² and about 25 mg/m², or between about 11 mg/m² and about 22 mg/m². In particular embodiments, the cationic liposomal formulation is administered at a dose of about 1 mg/m², about 2.5 mg/m², about 5 mg/m², about 7.5 mg/m², 11 mg/m², about 22 mg/m², about 25 mg/m², about 28 mg/m², about 31 mg/m², about 33 mg/m², about 35 mg/m², about 38 mg/m², about 41 mg/m², about 44 mg/m², or about 47 mg/m².

In embodiments, the cationic liposomal formulation is administered once or twice a week at a dose of about 11 mg/m² or about 22 mg/m². In other embodiments, the cationic liposomal formulation is administered on days 1, 8, and 15 of a 28-day treatment cycle. The treatment cycles may be repeated several times if desired, e.g. at least 2, 3, 4, 5, or 6 times.

The use in the methods disclosed herein includes administering a cationic liposomal formulation, a non-liposomal formulation including the taxane paclitaxel or a derivative thereof, and a further active agent gemcitabine. In particular embodiments, the cationic liposomal formulation used in the methods provided herein include DOTAP, DOPC, and paclitaxel.

In embodiments, a taxane paclitaxel in a single dose of the non-liposomal formulation is between about 5 mg/m² and about 100 mg/m², between about 5 mg/m² and about 50 mg/m², between about 50 mg/m² and about 100 mg/m², or between about 70 mg/m² and about 90 mg/m² body surface (bs) of a subject. In particular embodiments, the taxane in the non-liposomal formulation administered at about 70 mg/m², about 80 mg/m², or about 90 mg/m² bs of a subject and is administered on days 1, 8, and 15 of a 28-day treatment cycle. The treatment cycles may be repeated several times if desired, e.g. at least 2, 3, 4, 5 or 6 times.

In embodiments, gemcitabine is between about 100 mg/m² and about 1250 mg/m², between about 100 mg/m² and about 500 mg/m², between about 500 mg/m² and about 1250 mg/m², between about 600 mg/m² and about 1250 mg/m², about 700 mg/m² and about 1250 mg/m², or about 800 mg/m² and about 1250 mg/m² bs of a subject. In particular embodiments, gemcitabine is administered at a dose of about 800 mg/m², 1000 mg/m² or about 1250 mg/m² on days 1 and 8 of a 28-day treatment cycle. The treatment cycles may be repeated several times if desired, e.g. at least at least 2, 3, 4, 5 or 6 times. Gemcitabine (Gemzar®) is commercially available.

In 2004, the Food and drug Administration (FDA) approved administering gemcitabine (1250 mg/m² on days 1 and 8) in combination with paclitaxel (175 mg/m² on day 1) every 21 days for the first-line treatments of patients with metastatic breast cancer. In embodiments of the combination therapy provided herein, gemcitabine can be applied at a lower weekly dose compared to that in the FDA-approved standard therapy. In particular embodiments, gemcitabine may be administered at a weekly dose of between about 800 mg/m² to about 1000 mg/m².

In embodiments of the combination therapy provided herein, total paclitaxel (paclitaxel in cationic liposomal formulation and non-liposomal formulation) can be applied at a lower weekly dose compared to that in the FDA-approved standard therapy. In particular embodiments, the cationic liposomal formulation is administered at a dose of about 11 mg/m² or 22 mg/m² of paclitaxel in combination with a non-liposomal formulation at a dose of about 70 mg/m², about 80 mg/m², or about 90 mg/m² of paclitaxel, i.e. 81 mg/m², 91 mg/m², 92 mg/m², 101 mg/m², 102 mg/m² or 112 mg/m² of total dose of paclitaxel.

Cationic liposomal preparation are described which include 25-35 mg/m² of docetaxel. These doses are in amounts of mg/m² bs of a subject.

In embodiments, the cationic liposomal formulation is administered to a subject at a total monthly dose of between about 10 mg/m² and about 200 mg/m², about 20 mg/m² and about 150 mg/m², about 30 mg/m² and about 135 mg/m², about 40 mg/m² and about 120 mg/m², or about 60 mg/m² and about 100 mg/m² body surface (bs) of the subject. In particular embodiments, the cationic liposomal formulation is administered to the subject at a total monthly dose of about 11 mg/m², about 22 mg/m², about 33 mg/m², about 44 mg/m², about 66 mg/m², about 88 mg/m², about 110 mg/m², or about 132 mg/m² bs of the subject.

The doses of formulations can be administered a plurality of times daily to a plurality of times during a month period, each of the times being separated by a dosing interval of between days or weeks. In embodiments, a dosing interval can be 6 to 10 days.

The formulations are also suitable for long-term administration for one month, two months, three months, four months, five months, six months, seven months, eight months, nine months, ten months, eleven months, or twelve months. In embodiments, the duration of the administration of the once or twice weekly dosing schedule is several weeks, for example at least seven weeks.

The continued administration of lower doses once or twice weekly is at least as effective as the administration of a single high dose or frequent low dose administration interrupted by pause intervals. Depending on the effectiveness of the dosage regimen, the doses of the formulations and the dosing intervals may remain constant, increased, or decreased during the treatment period.

In embodiments, the cationic liposomal formulation and the non-liposomal formulation containing paclitaxel are administered once a week, on day 1, 8, and 15 of a treatment cycle, and gemcitabine is administered once a week, on day 1 and 8. The treatment free interval can be 6 to 14 days. In particular embodiments, the treatment cycle can be repeated for 6 to 12 times.

The cationic liposomal formulation or non-liposomal formulation can be administered intravenously.

The methods disclosed herein are characterized by selective targeting, improved efficacy, reduced adverse side effects as compared to conventional chemotherapy, reduced disease related pain, improved quality of life, stabilization of body weight during treatment, and synergistic effects with other therapy regimes.

Particularly, the once or twice weekly dosing schedule provided herein is less burdensome physically for the subject due to longer recovery times and fewer hospitalization. Moreover, the administration over a longer time frame of several weeks or months, for example 4 weeks to 52 weeks, is more efficacious than frequent applications over a short period. The once or twice weekly dosing schedule provides improved quality of life for the subject being treated.

As will be understood by one of ordinary skill in the art, each embodiment disclosed herein can comprise, consist essentially of or consist of its particular stated element, step, ingredient or component. Thus, the terms "include" or "including" should be interpreted to recite: "comprise, consist of, or consist essentially of." The transition term "comprise" or "comprises" means includes, but is not limited to, and allows for the inclusion of unspecified elements, steps, ingredients, or components, even in major amounts. The transitional phrase "consisting of' excludes any element, step, ingredient or component not specified. The transition phrase "consisting essentially of' limits the scope of the embodiment to the specified elements, steps, ingredients or components and to those that do not materially affect the embodiment, for example, a lack of a statistically-significant reduction in the ability to kill breast cancer cells in vitro or in vivo.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

As an example, the term "about" in the context of amount values refers to an average deviation of maximum +/-20%, +/-10%, or +/-5% based on the indicated value. For example, an amount of about 30 mole % cationic lipid refers to 30 mole %+/-6 mole %, or 30 mole %+/-3 mole % cationic lipid with respect to the total lipid/amphiphile molarity.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the disclosure.

Certain embodiments of this disclosure are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

The following examples illustrate exemplary methods provided herein.

### Examples of Embodiments

1. A method of treating breast cancer, wherein the method comprises administering to a subject in need thereof (a) a cationic liposomal formulation comprising one or more cationic lipids and a therapeutically effective amount of taxane; (b) a non-liposomal formulation comprising a therapeutically effective amount of taxane; and (c) a therapeutically effective amount of a further active agent, wherein the taxane is paclitaxel and the further active agent is gemcitabine.
2. The method of embodiment 1, wherein the method comprises administering to the subject a cationic liposomal formulation comprising about 1 mg/m² to about 60 mg/m² of the taxane; a non-liposomal formulation comprising about 5 mg/m² to about 100 mg/m² of the taxane in the non-liposomal formulation; and about 100 mg/m² to about 1250 mg/m² of the further active agent.
3. The method of embodiment 1 or 2, wherein the method comprises administering to the subject a cationic liposomal formulation comprising about 11 mg/m² to about 22 mg/m² of the taxane; a non-liposomal formulation comprising about 70 mg/m² to about 90 mg/m² taxane; and about 800 mg/m² to about 1250 mg/m² of the further active agent.
4. The method of any one of embodiments 1 to 3, wherein total dose of taxane in the cationic liposomal formulation and the non-liposomal formulation is between about 80 mg/m² and 120 mg/m².
5 The method of any one of embodiments 1 to 4, wherein the cationic liposomal formulation and the non-liposomal formulation are administered on days 1, 8, and 15 of a 28-day treatment cycle, and the further active agent is administered on days 1 and 8 of a 28-day treatment cycle.
6. The method of any one of embodiments 1 to 5, wherein the cationic liposomal formulation is administered first; the non-liposomal formulation is administered second; and the further active agent is administered third.
7. The method any one of embodiments 1 to 6, wherein the cationic liposomal formulation is administered to the subject at a rate of 0.5 mL/min for first 15 minutes, followed by a rate of 1.0 mL/min for second 15 minutes, and followed by a rate of 1.5 mL/min after 30 minutes.
8. The method of any one of embodiments 1 to 7, wherein the cationic liposomal formulation comprises a cationic lipid from about 30 mole% to about 99.9 mole%.
9. The method of any one of embodiments 1 to 8, wherein the cationic liposomal formulation comprises a taxane in an amount of at least 0.1 mole%.
10. The method of any one of embodiments 1 to 9, wherein the cationic liposomal formulation comprises a neutral and/or anionic lipid.
11. The method of any one of embodiments 1 to 10, wherein the cationic liposomal formulation further comprises a neutral or an anionic lipid in an amount of 30 mole% to 55 mole%.
12. The method of any one of embodiments 1 to 11, wherein the cationic liposomal formulation has a positive zeta potential in about 0.05 M KCI solution at about pH 7.5 at room temperature.
13. The method of any one of embodiments 1 to 12, wherein the taxane is paclitaxel.
14. The method of any one of embodiments 1 to 13, wherein the cationic liposomal formulation comprises DOTAP, DOPC, and paclitaxel.
15. The method of any one of embodiments 1-14, wherein the cationic liposomal formulation comprises DOTAP, DOPC, and paclitaxel in a mole ratio of about 50:47:3.
16. The method of any one of embodiments 1 to 15, wherein hormone receptor status of the subject is negative for both estrogen receptor and progesterone receptor, and the breast cancer is HER2 positive (HER2+).
17. The method of any one of embodiments 1 to 16, wherein the hormone receptor status of the subject is negative for both estrogen receptor and progesterone receptor, and the breast cancer is HER2 negative (HER2-).
18. The method of any one of embodiments 1 to 17, wherein the cationic lipid is N-[1-(2,3-dioleoyloxy)propyl]-N,N,N-trimethyl ammonium salt (DOTAP); dimethyldioctadecyl ammonium bromide (DDAB); 1,2-diacyloxy-3-trimethylammonium propane N-[1-(2,3-dioloyloxy)propyl]-N,N-dimethyl amine (DODAP); 1,2-diacyloxy-3-dimethylammonium propane; N-[1-(2,3-dioleyloxy)propyl]-N,N,N-trimethylammonium chloride (DOTMA); 1,2-dialkyloxy-3-dimethylammonium propane; dioctadecylamidoglycylspermine (DOGS); 3β-[N-(N',N'-dimethylamino-ethane)carbamoyl]cholesterol (DC-Chol); 2, 3-dioleoyloxy-N-(2-(sperminecarboxamido)-ethyl)-N, N-dimethyl-1-propanaminium trifluoroacetate (DOSPA); β-alanyl cholesterol; cetyl trimethyl ammonium bromide (CTAB); diC14-amidine; N-tert-butyl-N'-tetradecyl-3-tetradecylamino-propionamidine; 14Dea2; N-(alpha-trimethylammonioacetyl)didodecyl-D-glutamate chloride (TMAG); O,O'-ditetradecanoyl-N-(trimethylammonioacetyl)diethanolamine chloride; 1,3-dioleoyloxy-2-(6-carboxy-spermyl)-propylamide (DOSPER); N,N,N',N'-tetramethyl-N,N'-bis(2-hydroxylethyl)-2,3-dioleoyloxy-1,4-butanediammonium iodide; 1-[2-(acyloxy)ethyl]2-alkyl (alkenyl)-3-(2-hydroxyethyl)-imidazolinium chloride; 1,2-dioleoyl-3-dimethyl-hydroxyethylammonium bromide (DORI); 1,2-dioleyloxypropyl-3-dimethylhydroxyethylammonium bromide (DORIE); 1,2-dioleyloxypropyl-3-dimethylhydroxypropylammonium bromide (DORIE-HP); 1,2-dioleyloxypropyl-3-dimethylhydroxybutylammonium bromide (DORIE-HS); 1,2-dioleyloxypropyl-3-dimethylhydroxypentylammonium bromide (DORIE-Hpe); 1,2-dimyristyloxypropyl-3-dimethylhydroxylethylammonium bromide (DMRIE); 1,2-dipalmityloxypropyl-3-dimethylhydroxyethylammonium bromide (DPRIE); 1,2-disteryloxypropyl-3-dimethylhydroxyethylammonium bromide (DSRIE); or 1,2-diacyl-sn-glycerol-3-ethylphosphocholine.
19. The method of any one of embodiments 1 to 18, wherein the 1-[2-(acyloxy)ethyl]2-alkyl (alkenyl)-3-(2-hydroxyethyl)-imidazolinium chloride is 1-[2-(9(Z)-octadecenoyloxy)ethyl]-2-(8(Z)-heptadecenyl-3-(2-hydroxyethyl)-imidazoliniume hloride (DOTIM) or 1-[2-(hexadecanoyloxy)ethyl]-2-pentadecyl-3-(2-hydroxyethyl)imidazolinium chloride (DPTIM).
20. The method of any one of embodiments 1 to 19, wherein the neutral lipid is cholesterol, phospholipid, lysolipid, sphingolipid, or pegylated lipid with a neutral charge.
21. The method of any one of embodiments 1 to 20, wherein the neutral lipid is lysophospholipid.
22. The method of any one of embodiments 1 to 21, wherein the neutral lipid is 1,2-diacyl-sn-glycero-3-phosphoethanolamine, 1,2-diacyl-sn-glycero-3-phosphocholine, or sphingomyelin.
23. The method of any one of embodiments 1 to 22, wherein 1,2-diacyl-sn-glycero-3-phosphoethanolamine is 1,2-dioleoyl-sn-glycero-3-phosphoethanolamine (DOPE).
24. The method of any one of embodiments 1 to 22, wherein 1,2-diacyl-sn-glycero-3-phosphocholine is 1,2-dioleoyl-sn-glycero-3-phosphocholine (DOPC).
25. The method of any one of embodiments 1 to 24, wherein the further active agent is gemcitabine.

### EXAMPLES

### Example 1: Activity of drug combinations in a TNBC Tumor Model

The anti-tumor activity was assessed in an *in vitro* human metastasis model with a TNBC cell line, MDMAB-231. MDMAB-231 was plated at 4000 cells per well in 96-well plates and incubated at 37°C. The cells were treated with drug combinations on the next day. The anti-growth effect of the drug combinations on the cells was measured by MTS/PMS cell viability assay (MTS:3-(4,5-dimethylthiazol-2-yl)-5-(3-carboxymethoxyphenyl)-2-(4-sulfophenyl)-2H-tetrazolium, inner salt; PMS: phenazine methosulfate).

. The metabolic activity was used to estimate individual IC₅₀ value by using a nonlinear regression analysis. All experiments were performed in triplicate.

Drug Combination: Gemcitabine + Taxol®:
MDMAB-231 cells were plated at 4000 cells/well in 96-well plates and incubated at 37°C/5% CO₂ for 16 hours, and then treated with Taxol® (paclitaxel) at a concentration of 600 nM, 200 nM, 100 nM, 50 nM, 20 nM, 10 nM, 5 nM or 1 nM in the culture medium. After 24 hours, gemcitabine in 10-fold or 5-fold concentration relative to the concentration of paclitaxel was added. For example, 1 nM paclitaxel was combined with 10 nM or 5 nM gemcitabine. After a 72-hour incubation period, the growth-inhibitory effect of gemcitabine and Taxol® on the cell line was measured by MTS/PMS cell viability assay.

Drug Combination: Gemcitabine + Taxol® + EndoTAG®-1
MDMAB-231 cells were plated at 4000 cells/well in 96-well plates and incubated at 37°C/5% CO₂ for 16 hours, and then treated with Taxol® and EndoTAG®-1. The mole ratio of Taxol® and EndoTAG®-1 was 1:1, and the total Taxol® concentration was 600 nM, 200 nM, 100 nM, 50 nM, 20 nM, 10 nM, 5 nM or 1 nM in culture medium. After 24 hours, gemcitabine in 10-fold or 5-fold concentration relative to the concentration of total paclitaxel was added. For example, 1 nM paclitaxel was combined with 10 nM or 5 nM gemcitabine. After a 72-hour incubation period, the growth-inhibitory effect of gemcitabine and Taxol® and EndoTAG®-1 was measured by MTS/PMS cell viability assay.

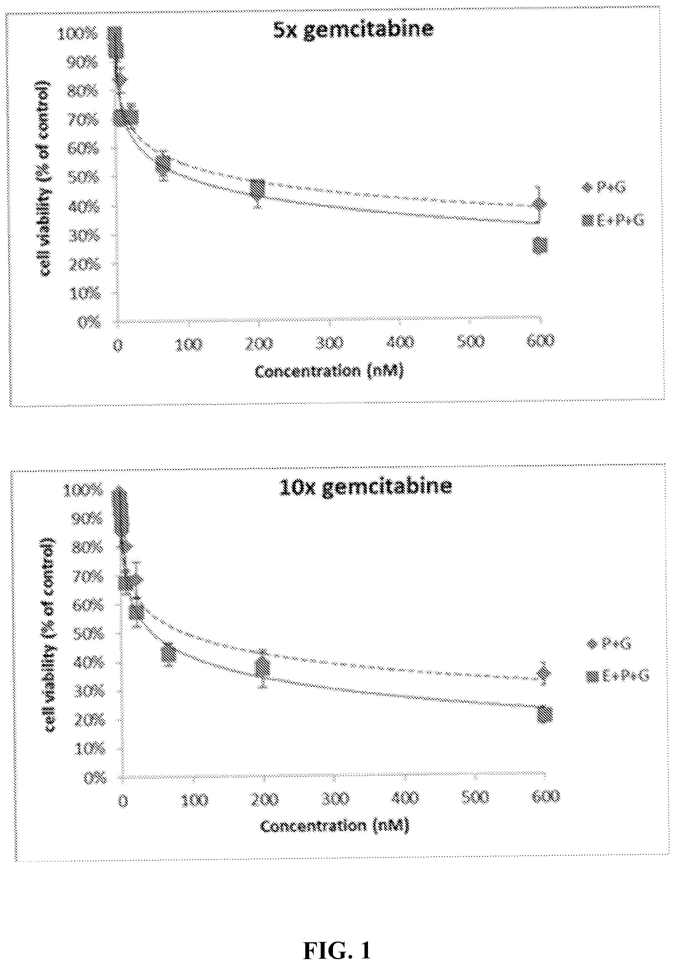

As shown in FIG. 1, EndoTAG®-1 + Taxol® + gemcitabine (triple combination) showed enhanced anti-tumor activity than Taxol® + gemcitabine (double combination). The IC₅₀ of Taxol® in the double combination and triple combination was 139.2 nM and 100.4 nM, respectively.

### Example 2: Evaluating the Efficacy of EndoTAG®-1 in Triple Receptor Negative Breast Cancer (TNBC) Patients

### 2.1 Objectives

The primary objective is to compare efficacy of weekly infusions of EndoTAG®-1 containing paclitaxel in combination with non-liposomal paclitaxel and gemcitabine versus weekly infusions of non-liposomal paclitaxel in combination with gemcitabine in patients with metastatic TNBC. The secondary objective is to obtain data on efficacy as well as data on safety and tolerability of weekly infusions of EndoTAG®-1 containing paclitaxel in combination with non-liposomal paclitaxel and gemcitabine versus weekly infusions of non-liposomal paclitaxel in combination with gemcitabine in patients with metastatic TNBC.

### 2.2 Endpoints

### Primary Efficacy Endpoint:

Progression free survival (PFS) is defined as the time from randomization to disease progression based on blind central radiological image evaluation or death from any cause, whichever occurs first.

Secondary Efficacy Endpoints:
1. Overall survival (OS) is defined as the time from randomization to death from any cause.
2. Clinical benefit rate is defined as an objective response of any duration or stable disease lasting ≥ 6 months.
3. Best overall tumor response rate is defined as an objective response or stable disease of any duration.
4. Duration of response is defined as the time from randomization to disease progression in the subgroup of patients responding to therapy.
5. Biomarker analysis: serum tumor markers: CA15.3, CEA and CYFRA 21.1; and additional biomarkers: circulating tumor cells and tumor stem cells circulating endothelial and endothelial precursor cells, VEGF.

### 2.3 Study Design

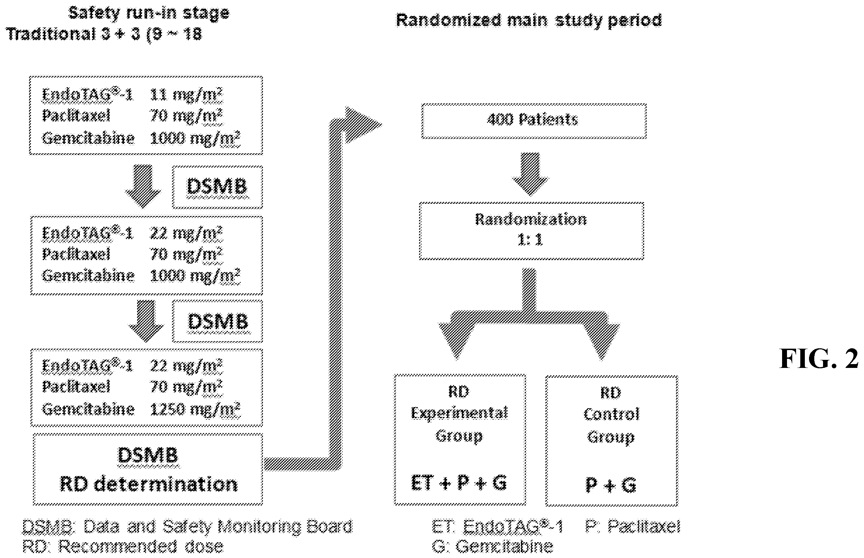

There are two stages for this study, a dose escalating safety run-in stage; and a main study stage. A schematic diagram of the study design is shown in FIG. 2.

### 2.3.1 Safety run-in Stage

Prior to the commencement of main study, there is a safety run-in stage to determine the recommended dose (RD) for main study. During the dose-escalating safety run-in stage, 3 dose-dense weekly taxane regimens are administered to approximately 9∼18 patients. Each treatment cycle comprises 3 weeks of treatment followed by a 1-week treatment-free interval for a total of 4 weeks. A data and safety monitoring board (DSMB) is instituted to decide on the RD for the EndoTAG®-1, the non-liposomal paclitaxel, and gemcitabine for use throughout the trial and for monitoring the patient's safety and treatment efficacy data.

2.3.1.1 Experimental groups:
Dose 1: Intravenous infusions of 11 mg/m² of paclitaxel in EndoTAG®-1 and 70 mg/m² of non-liposomal paclitaxel on days 1, 8, and 15, and 1000 mg/m² gemcitabine on days 1 and 8 of a 28-day cycle.
Dose 2: Intravenous infusions of 22 mg/m² of paclitaxel in EndoTAG®-1 and 70 mg/m² of a non-liposomal paclitaxel on days 1, 8, and 15, and 1000 mg/m² gemcitabine on days 1 and 8 of a 28-day cycle.
Dose 3: Intravenous infusions of 22 mg/m² of paclitaxel in EndoTAG®-1 and 70 mg/m² of a non-liposomal paclitaxel on days 1, 8, and 15, and 1250 mg/m² gemcitabine on days 1 and 8 of a 28-day cycle.

During the safety run-in stage, patients are treated by the above mentioned dose cohorts starting from Dose 1. Dose cohorts follow one another sequentially based on the decision of DSMB. Accumulated data of the first treatment cycle are reviewed. Escalation to the next dose level proceeds after the DSMB reviewed safety data of the current cohort. The dose is escalated if the DSMB does not identify significant safety and tolerability concerns after reviewing safety data, especially for pre-defined dose limiting toxicity (DLT). Based on the results from the safety run-in stage, the DSMB defines the RD for the main study.

### 2.3.2 Main Study

In the main study, a total of 400 patients is randomized to treatment or control group in a 1:1 allocation ratio. Eligible patients are treated with EndoTAG®-1, a non-liposomal paclitaxel and gemcitabine at RD or doses based on the following control groups:

Doses in Control Groups to be selected after RD Determination:
- Control for Dose 1: Intravenous infusions of 80 mg/m² paclitaxel on days 1, 8, and 15 and 1000 mg/m² gemcitabine on days 1 and 8 of a 28-day cycle
- Control for Dose 2: Intravenous infusions of 90 mg/m² paclitaxel on days 1, 8, and 15 and 1000 mg/m² gemcitabine on days 1 and 8 of a 28-day cycle
- Control for Dose 3: Intravenous infusions of 90 mg/m² paclitaxel on days 1, 8, and 15 and 1250 mg/m² gemcitabine on days 1 and 8 of a 28-day cycle

### 2.4 Selection of study population

### Inclusion Criteria

Patients who meet the following criteria are considered eligible to participate in the study:
1. Gender: Female
2. Age ≥ 18 years or legal age to provide informed consent according to local regulatory requirements.
3. Metastatic TNBC confirmed histologically by a certified local laboratory (or existing medical record for confirmation is acceptable for patients in the safety run-in stage) using archival paraffinated material from the original surgery specimens or from later materials, as available. Results of the certified local laboratory must be available to allow for randomization. *Tumors should be considered negative for ER and PrR by immunohistochemistry (IHC; < 1% positive tumor nuclei, as per ASCO*/*College of American Pathologists (CAP) guideline recommendations, Hammond et al 2010) and negative for HER2 by IHC or fluorescent or chromogenic in situ hybridization (FISH or CISH). Patients with equivocal HER2 results by IHC should have the negativity status confirmed by FISH.*
4. Patients must have had prior adjuvant treatment with either sequential or concurrent anthracycline- and/or taxane-based chemotherapy.
5. Patients with a disease-free interval (DFI) on anthracycline- and/or taxane-based adjuvant therapy of > 12 months.
6. Patients must be indicated for treatment with polychemotherapy for visceral metastatic disease as judged by the Investigator.
7. At least one measurable or non-measurable tumor lesion according to RECIST version 1.1 as assessed by the Investigator (local radiological image assessment).
8. ECOG performance status 0 or 1.
9. Negative pregnancy test (females of childbearing potential).
10. Willingness to perform double-barrier-contraception during study and for 6 months post chemotherapy treatment (females of childbearing potential)
11. Signed informed consent.

### Exclusion Criteria

Patients who meet one or more of the following criteria will not be considered eligible to participate in the study:
1. Prior first-line chemotherapy for locally recurrent and/or metastatic breast cancer, including visceral disease.
2. Brain metastasis/known progressive cerebral metastasis (patients with cerebral metastases in a stable state or after successful surgical or radiological treatment are allowed to participate in the study).
3. Major surgery < 4 weeks prior to enrollment.
4. Cancer immunotherapy at any time.
5. Severe pulmonary obstructive or restrictive disease.
6. Uncontrolled inflammatory disease (autoimmune or infectious).
7. Clinically significant cardiac disease (New York Heart Association [NYHA] stadium > 2)
8. Results of laboratory tests (hematology, coagulation, clinical chemistry) outside specified limits:
   - White blood cell (WBC) count ≤ 3 × 10⁹/L
   - Absolute neutrophil count (ANC) ≤ 1.5 × 10⁹/L
   - Platelets ≤ 100 × 10⁹/L
   - Hemoglobin (Hb) ≤ 9.0 g/dL (≤ 5.6 mmol/L)
   - Partial thromboplastin time/international normalized ratio (PTT/INR) > 1.5 × upper limit of normal (ULN)
   - Aspartate aminotransferase (AST) or alanine aminotransferase (ALT) > 2.5 × ULN (> 5 x ULN if presence of liver metastasis)
   - Alkaline phosphatase (AP) > 2 × ULN (> 5 × ULN if presence of liver metastasis)
   - Total bilirubin > 1.5 × ULN (> 2.5 × ULN if presence of liver metastasis)
9. Pregnancy or nursing status.
10. Known positive human immunodeficiency virus (HIV) infection in medical history.
11. Peripheral neuropathy associated to prior taxane therapy not recovered to grade 0 or 1.
12. Known hypersensitivity to any component of the EndoTAG®-1, standard paclitaxel and/or gemcitabine formulations.
13. History of malignancy other than breast cancer < 5 years prior to enrollment, except skin cancer (i.e., basal or squamous cell carcinoma) treated locally.
14. History of active or significant neurological disorder or psychiatric disorder that would prohibit the understanding and giving of informed consent, or would interfere in the clinical and radiological evaluation of the central nervous system during the trial.
15. Concurrent treatment with other experimental products. Participation in another clinical trial with any investigational product within 30 days prior to study entry.
16. Positive test for hepatitis B (hepatitis B virus surface antigen (HBsAg) positive; or HBsAg negative but anti-hepatitis B virus core (HBc) antibody positive and HBV DNA positive) or hepatitis C (anti-hepatitis C virus (HCV) antibody positive). Patients that are anti-HCV antibody positive can also be judged eligible if further HCV RNA detection shows negative results.

### 2.5 Drug administration

For patients to be administered with EndoTAG®-1/paclitaxel/gemcitabine, whether in the safety run-in stage or main study, the dose of EndoTAG®-1 is administered prior to paclitaxel and gemcitabine (days 1 and 8) or prior to paclitaxel (day 15) on the same day. Infusion of EndoTAG®-1 should be started slowly (15 min at 0.5 mL/min, followed by 15 min at 1.0 mL/min) and increased to a maximum speed of 1.5 mL/min. Infusion of paclitaxel should not be started within 1 hour after the end of EndoTAG®-1 infusion. Paclitaxel should be administered as a 1-hour infusion (90 mg/m²) or less (doses below 90 mg/m²). Following paclitaxel infusion, gemcitabine should be administered intravenously over 30 minutes.

In all treatment groups, the standard premedication for paclitaxel (i.e., intravenous infusion of 10 mg dexamethasone) is administered 30 to 60 minutes prior to each infusion of paclitaxel.

### 2.6 Dose adjustment

The dose and schedule of study treatment may be modified (generally reduced) based on toxicity. Classification and severity grading of toxicities are assessed using the NCI-CTCAE version 4.03.

In the safety run-in stage, if specific AE occurs in patients and the Investigators consider necessary, dose reductions are executed according to the following regimen:

**Dose Reduction for Grade 4 Non-Neurotoxicity (including Bone Marrow toxicity)**

| Treatment | | Dose reduction 1 | Dose reduction 2 |
|---|---|---|---|
| Dose 1: | | | |
| EndoTAG®-1: | 11 mg/m² | 11 mg/m² | 11 mg/m² |
| Paclitaxel: | 70 mg/m² | 70 mg/m² | 60 mg/m² |
| Gemcitabine: | 1000 mg/m² | 800 mg/m² | 800 mg/m² |

| Dose 2: | | | |
|---|---|---|---|
| EndoTAG®-1: | 22 mg/m² | 22 mg/m² | 22 mg/m² |
| Paclitaxel: | 70 mg/m² | 70 mg/m² | 60 mg/m² |
| Gemcitabine: | 1000 mg/m² | 800 mg/m² | 800 mg/m² |

| Dose 3: | | | |
|---|---|---|---|
| EndoTAG®-1: | 22 mg/m² | 22 mg/m² | 22 mg/m² |
| Paclitaxel: | 70 mg/m² | 70 mg/m² | 70 mg/m² |
| Gemcitabine: | 1250 mg/m² | 1000 mg/m² | 800 mg/m² |

**Dose Reduction for Grade 4 Neurotoxicity**

| Treatment | | Dose reduction 1 | Dose reduction 2 |
|---|---|---|---|
| Dose 1: | | | |
| EndoTAG®-1: | 11 mg/m² | 11 mg/m² | 11 mg/m² |
| Paclitaxel: | 70 mg/m² | 60 mg/m² | 50 mg/m² |
| Gemcitabine: | 1000 mg/m² | 1000 mg/m² | 1000 mg/m² |

| Dose 2: | | | |
|---|---|---|---|
| EndoTAG®-1: | 22 mg/m² | 22 mg/m² | 22 mg/m² |
| Paclitaxel: | 70 mg/m² | 60 mg/m² | 50 mg/m² |
| Gemcitabine: | 1000 mg/m² | 1000 mg/m² | 1000 mg/m² |

| Dose 3: | | | |
|---|---|---|---|
| EndoTAG®-1: | 22 mg/m² | 22 mg/m² | 22 mg/m² |
| Paclitaxel: | 70 mg/m² | 60 mg/m² | 50 mg/m² |
| Gemcitabine: | 1250 mg/m² | 1250 mg/m² | 1250 mg/m² |

Transiently reduced doses are administered once, after which the dose will return to the regular dose level. If the same transient dose reduction is applied twice in a patient, the dose level is permanently reduced for this patient.

For patients in the main study, dose reduction due to safety issues is carried out when Investigator considers applicable. The regimen is determined by the Investigator based on previous experiences of dose reduction in the safety run-in stage.

### 2.7 General assessment

### Assessment of Tumor Receptor Status

In the screening stage of the main study, tissue samples of relapsed tumor, metastases, or initial tumor are sent to the certified local laboratory for histological confirmation of the triple-negative tumor receptor status. Instructions for shipment and shipment documents are provided in the ISF of each study site. Tumors are analyzed by IHC for expression of ER and PrR and by IHC or, FISH, or CISH for expression of HER2. Patients with equivocal HER2 results by IHC should have the negativity status confirmed by FISH.

To allow further classification of the patient population, tumors are analyzed by IHC for expression of EGFR and Cytokeratin 5/6 in addition to ER, PrR and HER2 expression (five-biomarker method to identify core basal-like tumors; Cheang et al 2008). Tumor tissues will also be analyzed for mutations of BRCA1, BRCA2 and p53 and expression levels of claudin 3/4 and claudin 7 and of the proliferation marker Ki-67.

### 2.8 Efficacy Assessments

Progression-free survival is defined as the time from randomization to disease progression or death from any cause, whichever occurs first. Post-progression PFS (PFS-2) is defined as the time from start of second and subsequent lines of therapy administered after trial participation to disease progression (based on local radiological image evaluation or clinical assessment) or death from any cause, whichever occurs first. Overall survival is defined as the time from randomization to death from any cause.

Tumor response is assessed at the study sites according to RECIST version 1.1. When more than one measurable lesion is present at baseline, all lesions up to a maximum of 5 lesions in total and a maximum of 2 lesions per organ, representative of all involved organs, should be identified as target lesions. Target lesions should be selected on the basis of their size (lesions with the longest diameter) and their suitability for accurate repeated measurements (either by imaging techniques or clinically). All measurements should be recorded in metric notation by use of a ruler or calipers. A sum of the diameters (longest for non-nodal lesions, short axis for nodal lesions) for all target lesions is calculated and reported. Evaluation of target lesions:
- Complete response (CR): The disappearance of all target lesions
- Partial response (PR): At least a 30% decrease in the sum of diameters of target lesions, taking as reference the baseline sum diameters
- Progressive disease (PD): At least a 20% increase in the sum of diameters of target lesions, taking as reference the smallest sum of diameters on study, and at least an increase by 5 mm. The appearance of one or more new lesions is also considered progression
- Stable disease (SD): Neither sufficient shrinkage to qualify for PR nor sufficient increase to qualify for PD, taking as reference the smallest sum of diameters while on study

All other lesions present at screening are identified as non-target lesions. Measurements of these lesions are not required, but the presence or absence of each should be noted. Evaluation of Non-target lesions:
- CR: The disappearance of all non-target lesions and normalization of tumor marker level
- Non-CR/non-PD: The persistence of one or more non-target lesion(s) and/or the maintenance of tumor marker level above the normal limits
- PD: Unequivocal progression of existing non-target lesions. The appearance of one or more new lesions is also considered progression

For assessment of the quality of life, the EORTC QLQ-C30- and BR23-Questionnaires are used from screening (baseline value) until disease progression in the main study. The questionnaires should be filled in by the patient at the beginning of each study visit prior to any study-related examinations or treatments.

## Claims

1. A combination comprising:
(a) a cationic liposomal formulation comprising one or more cationic lipids and a therapeutically effective amount of taxane;
(b) a non-liposomal formulation comprising a therapeutically effective amount of taxane; and
(c) a therapeutically effective amount of a further active agent,
for use in a method of treating breast cancer wherein the taxane is paclitaxel and the further active agent is gemcitabine.

2. The combination of claim 1 for use in a method of treating breast cancer, wherein the method comprises administering to the subject a cationic liposomal formulation comprising 1 mg/m² to 60 mg/m² of the taxane; a non-liposomal formulation comprising 5 mg/m² to 100 mg/m² of the taxane in the non-liposomal formulation; and 100 mg/m² to 1250 mg/m² of the further active agent, and wherein the method particularly comprises administering to the subject a cationic liposomal formulation comprising 11 mg/m² to 22 mg/m² of the taxane; a non-liposomal formulation comprising 70 mg/m² to 90 mg/m² taxane; and 800 mg/m² to 1250 mg/m² of the further active agent.

3. The combination of claim 1 or 2 for use in a method of treating breast cancer, wherein the total dose of taxane in the cationic liposomal formulation and the non-liposomal formulation is between 80 mg/m² and 120 mg/m².

4. The combination of claim 1 or 2 for use in a method of treating breast cancer, wherein the cationic liposomal formulation and the non-liposomal formulation are administered on days 1, 8, and 15 of a 28-day treatment cycle, and the further active agent is administered on days 1 and 8 of a 28-day treatment cycle, and/or wherein the cationic liposomal formulation is administered first; the non-liposomal formulation is administered second; and the further active agent is administered third.

5. The combination of claim 1 or 2 for use in a method of treating breast cancer, wherein the cationic liposomal formulation is administered to the subject at a rate of 0.5 mL/min for first 15 minutes, followed by a rate of 1.0 mL/min for second 15 minutes, and followed by a rate of 1.5 mL/min after 30 minutes.

6. The combination of claim 1 or 2 for use in a method of treating breast cancer, wherein the cationic liposomal formulation comprises a cationic lipid from 30 mole% to 99.9 mole% and/or wherein the cationic liposomal formulation comprises a taxane in an amount of at least 0.1 mole%.

7. The combination of claim 1 or 2 for use in a method of treating breast cancer, wherein the cationic liposomal formulation comprises a neutral and/or anionic lipid, wherein the cationic liposomal formulation particularly comprises a neutral or an anionic lipid in an amount of 30 mole% to 55 mole%, and wherein the neutral lipid may be cholesterol, phospholipid, lysolipid, sphingolipid, pegylated lipid with a neutral charge, or lysophospholipid, particularly 1,2-diacyl-sn-glycero-3-phosphoethanolamine such as 1,2-dioleoyl-sn-glycero-3-phosphoethanolamine (DOPE), 1,2-diacyl-sn-glycero-3-phosphocholine such as 1,2-dioleoyl-sn-glycero-3-phosphocholine (DOPC), or sphingomyelin.

8. The combination of claim 1 or 2 for use in a method of treating breast cancer, wherein the cationic liposomal formulation has a positive zeta potential in about 0.05 M KCI solution at about pH 7.5 at room temperature.

9. The combination of claim 1 or 2 for use in a method of treating breast cancer, wherein the cationic liposomal formulation comprises DOTAP, DOPC, and paclitaxel, and wherein the cationic liposomal formulation particularly comprises DOTAP, DOPC, and paclitaxel in a mole ratio of about 50:47:3.

10. The combination of claim 1 or 2 for use in a method of treating breast cancer, wherein hormone receptor status of the subject is negative for both estrogen receptor and progesterone receptor, and the breast cancer is HER2 positive (HER2+), or wherein the hormone receptor status of the subject is negative for both estrogen receptor and progesterone receptor, and the breast cancer is HER2 negative (HER2-).

11. The combination of claim 1 or 2 for use in a method of treating breast cancer, wherein the cationic lipid is N-[1-(2,3-dioleoyloxy)propyl]-N,N,N-trimethyl ammonium salt (DOTAP); dimethyldioctadecyl ammonium bromide (DDAB); 1,2-diacyloxy-3-trimethylammonium propane N-[1-(2,3-dioloyloxy)propyl]-N,N-dimethyl amine (DODAP); 1,2-diacyloxy-3-dimethylammonium propane; N-[1-(2,3-dioleyloxy)propyl]-N,N,N-trimethylammonium chloride (DOTMA); 1,2-dialkyloxy-3-dimethylammonium propane; dioctadecylamidoglycylspermine (DOGS); 3β-[N-(N',N'-dimethylamino-ethane)carbamoyl]cholesterol (DC-Chol); 2, 3-dioleoyloxy-N-(2-(sperminecarboxamido)-ethyl)-N, N-dimethyl-1-propanaminium trifluoroacetate (DOSPA); β-alanyl cholesterol; cetyl trimethyl ammonium bromide (CTAB); diC14-amidine; N-tert-butyl-N'-tetradecyl-3-tetradecylamino-propionamidine; 14Dea2; N-(alpha-trimethylammonioacetyl)didodecyl-D-glutamate chloride (TMAG); O,O'-ditetradecanoyl-N-(trimethylammonioacetyl)diethanolamine chloride; 1,3-dioleoyloxy-2-(6-carboxy-spermyl)-propylamide (DOSPER); N,N,N',N'-tetramethyl-N,N'-bis(2-hydroxylethyl)-2,3-dioleoyloxy-1,4-butanediammonium iodide; 1-[2-(acyloxy)ethyl]2-alkyl (alkenyl)-3-(2-hydroxyethyl)-imidazolinium chloride; 1,2-dioleoyl-3-dimethyl-hydroxyethylammonium bromide (DORI); 1,2-dioleyloxypropyl-3-dimethylhydroxyethylammonium bromide (DORIE); 1,2-dioleyloxypropyl-3-dimethylhydroxypropylammonium bromide (DORIE-HP); 1,2-dioleyloxypropyl-3-dimethylhydroxybutylammonium bromide (DORIE-HS); 1,2-dioleyloxypropyl-3-dimethylhydroxypentylammonium bromide (DORIE-Hpe); 1,2-dimyristyloxypropyl-3-dimethylhydroxylethylammonium bromide (DMRIE); 1,2-dipalmityloxypropyl-3-dimethylhydroxyethylammonium bromide (DPRIE); 1,2-disteryloxypropyl-3-dimethylhydroxyethylammonium bromide (DSRIE); or 1,2-diacyl-sn-glycerol-3-ethylphosphocholine, and wherein the 1-[2-(acyloxy)ethyl]2-alkyl (alkenyl)-3-(2-hydroxyethyl)-imidazolinium chloride is particularly 1-[2-(9(Z)-octadecenoyloxy)ethyl]-2-(8(Z)-heptadecenyl-3-(2-hydroxyethyl)-imidazoliniumchloride (DOTIM) or 1-[2-(hexadecanoyloxy)ethyl]-2-pentadecyl-3-(2-hydroxyethyl)imidazolinium chloride (DPTIM).

## Patentansprüche

1. Kombination umfassend:
(a) eine kationische liposomale Formulierung umfassend ein oder mehrere kationische Lipide und einen therapeutisch wirksamen Gehalt an Taxan
(b) eine nicht-liposomale Formulierung umfassend einen therapeutisch wirksamen Gehalt an Taxan; und
(c) einen therapeutisch wirksamen Gehalt eines weiteren Wirkstoffs, zur Verwendung in einem Verfahren zur Behandlung von Brustkrebs, wobei das Taxan Paclitaxel ist und der weitere Wirkstoff Gemcitabin ist.

2. Kombination nach Anspruch 1, zur Verwendung in einem Verfahren zur Behandlung von Brustkrebs, wobei das Verfahren die Verabreichung einer kationischen, liposomalen Formulierung, umfassend 1 mg/m² bis 60 mg/m² des Taxans; einer nicht-liposomalen Formulierung, umfassend 5 mg/m² bis 100 mg/m² des Taxans in der nicht-liposomalen Formulierung; und 100 mg/m² bis 1250 mg/m² des weiteren Wirkstoffs an das Subjekt umfasst, und wobei die Methode insbesondere die Verabreichung einer kationischen, liposomalen Formulierung, umfassend 11 mg/m² bis 22 mg/m² des Taxans; einer nicht-liposomalen Formulierung, umfassend 70 mg/m² bis 90 mg/m² Taxan; und 800 mg/m² bis 1250 mg/m² des weiteren Wirkstoffs an das Subjekt umfasst.

3. Kombination nach Anspruch 1 oder 2 zur Verwendung in einem Verfahren zur Behandlung von Brustkrebs, wobei die Gesamtdosis des Taxans in der kationischen, liposomalen Formulierung und der nicht-liposomalen Formulierung zwischen 80 mg/m² und 120 mg/m² ist.

4. Kombination nach Anspruch 1 oder 2 zur Verwendung in einem Verfahren zur Behandlung von Brustkrebs, wobei die kationische liposomale Formulierung und die nicht-liposomale Formulierung an Tag 1, 8, und 15 eines 28 tägigen Behandlungszyklus verabreicht werden und der weitere Wirkstoff an Tag 1 und 8 eines 28 tägigen Behandlungszyklus verabreicht wird, und/oder wobei die kationische liposomale Formulierung zuerst verabreicht wird; die nicht-liposomale Formulierung als Zweites verabreicht wird; und der weitere Wirkstoff als Drittes verabreicht wird.

5. Kombination nach Anspruch 1 oder 2 zur Verwendung in einem Verfahren zur Behandlung von Brustkrebs, wobei die kationische liposomale Formulierung dem Subjekt mit einer Rate von 0,5 ml/min für die ersten 15 Minuten, gefolgt von einer Rate von 1,0 ml/min für die zweiten 15 Minuten, und gefolgt von einer Rate von 1,5 ml/min nach 30 Minuten, verabreicht wird.

6. Kombination nach Anspruch 1 oder 2 zur Verwendung in einem Verfahren zur Behandlung von Brustkrebs, wobei die kationische liposomale Formulierung ein kationisches Lipid von 30 mol% bis 99,9 mol% umfasst und/oder wobei die kationische liposomale Formulierung ein Taxan in einem Gehalt von wenigstens 0,1 mol% umfasst.

7. Kombination nach Anspruch 1 oder 2 zur Verwendung in einem Verfahren zur Behandlung von Brustkrebs, wobei die kationische liposomale Fromulierung ein neutrales und/oder anionisches Lipid umfasst, wobei die kationische liposomale Formulierung insbesondere ein neutrales oder anionisches Lipid in einem Gehalt von 30 mol% bis 55 mol% umfasst, und worin das neutrale Lipid Cholesterol, Phospholipid, Lysolipid, Sphingolipid, pegyliertes Lipid mit neutraler Ladung, oder Lysophospholipid, insbesondere 1,2-Diacyl-sn-glycero-3-phosphoethanolamin wie etwa 1,2-Dioleoyl-sn-glycero-3-phosphoethanolamin (DOPE), 1,2-Diacyl-sn-glycero-3-phosphocholin wie etwa 1,2-Dioleoyl-sn-glycero-3-phosphocholin (DOPC), oder Sphingomyelin umfasst.

8. Kombination nach Anspruch 1 oder 2 zur Verwendung in einem Verfahren zur Behandlung von Brustkrebs, wobei die kationische liposomale Formulierung in einer etwa 0,05 M KCl Lösung bei etwa pH 7,5 bei Raumtemperatur ein positives Zetapotential aufweist.

9. Kombination nach Anspruch 1 oder 2 zur Verwendung in einem Verfahren zur Behandlung von Brustkrebs, wobei die kationische liposomale Formulierung DOTAP, DOPC und Paclitaxel umfasst und wobei die kationische liposomale Formulierung insbesondere DOTAP, DOPC und Paclitaxel in einem molaren Verhältnis von etwa 50:47:3 umfasst.

10. Kombination nach Anspruch 1 oder 2 zur Verwendung in einem Verfahren zur Behandlung von Brustkrebs, wobei der Hormonrezeptor Status des Subjekts sowohl für den Östrogenrezeptor als auch den Progestronrezeptor negativ ist und der Brustkrebs HER2 positiv (HER2+) ist, oder wobei der Hormonrezeptor Status sowohl für den Östrogenrezeptor als auch den Progestronrezeptor negativ ist und der Brustkrebs HER2 negativ (HER2-) ist.

11. Kombination nach Anspruch 1 oder 2 zur Verwendung in einem Verfahren zur Behandlung von Brustkrebs, wobei das kationische Lipid N-[1-(2,3-Dioleoyloxy)propyl]-N,N,N-trimethylammoniumsalz (DOATP); Dimethyldioctadecyl Ammoniumbromid (DDAB); 1,2-Diacyloxy-3-trimethylammoniumpropan N-[1-(2,3-Dioloyloxy)propyl]-N,N-dimethylamin (DODAP); 1,2-Diacyloxy-3-dimethylammoniumpropan; N-[1-(2,3-Dioleyloxy)propyl]-N,N,N-trimethylammoniumchlorid (DOTMA); 1,2-Dialkyloxy-3-dimethylammoniumpropan; Dioctadecylamidoglycylspermin (DOGS); 3β-[N-(N',N'-Dimethylamino-ethan)carbamoyl]cholesterin (DC-Chol); 2,3-Dioleoyloxy-N-(2-(sperminecarboxamido)-ethyl)-N, N-dimethyl-1-propanaminiumtrifluoracetat (DOSPA); β-Alanylcholesterin; Cetyltrimethylammoniumbromid (CTAB); diC14-Amidin; N-tert-Butyl-N'-tetradecyl-3-tetradecylamino-propionamidin; 14Dea2; N-(alpha-Trimethylammonioacetyl)didodecyl-D-glutamatchlorid (TMAG); O,O'-Ditetradecanoyl-N-(trimethylammonioacetyl)diethanolaminchlorid; 1,3-Dioleoyloxy-2-(6-carboxy-spermyl)-propylamid (DOSPER); N,N,N',N'-Tetramethyl-N,N'-bis(2-hydroxylethyl)-2,3-dioleoyloxy-1,4-butandiammoniumjodid; 1-[2-(Acyloxy)ethyl]2-alkyl(alkenyl)-3-(2-hydroxyethyl)-imidazoliniumchlorid; 1,2-Dioleoyl-3-dimethyl-hydroxyethylammoniumbromid (DORI); 1,2-Dioleyloxypropyl-3-dimethylhydroxyethylammoniumbromid (DORIE); 1,2-Dioleyloxypropyl-3-dimethylhydroxypropylammoniumbromid (DORIE-HP); 1,2-Dioleyloxypropyl-3-dimethylhydroxybutylammoniumbromid (DORIE-HS); 1,2-Dioleyloxypropyl-3-dimethylhydroxypentylammoniumbromid (DORIE-Hpe); 1,2-Dimyristyloxypropyl-3-dimethylhydroxylethylammoniumbromid (DMRIE); 1,2-Dipalmityloxypropyl-3-dimethylhydroxyethylammoniumbromid (DPRIE); 1,2-Disteryloxypropyl-3-dimethylhydroxyethylammoniumbromid (DSRIE); oder 1,2-Diacyl-sn-glycerol-3-ethylphosphocholin ist, und wobei das 1-[2-(Acyloxy)ethyl]2-alkyl (alkenyl)-3-(2-hydroxyethyl)-imidazoliniumchlorid insbesondere 1-[2-(9(Z)-Octadecenoyloxy)ethyl]-2-(8(Z)-heptadecenyl-3-(2-hydroxyethyl)-imidazoliniumchlorid (DOTIM) oder 1-[2-(Hexadecanoyloxy)ethyl]-2-pentadecyl-3-(2-hydroxyethyl)imidazoliniumchlorid (DPTIM) ist.

## Revendications

1. Combinaison comprenant:
(a) une formulation liposomale cationique comprenant un ou plusieurs lipides cationiques et une quantité thérapeutiquement efficace de taxane;
(b) une formulation non liposomale comprenant une quantité thérapeutiquement efficace de taxane; et
(c) une quantité thérapeutiquement efficace d'un autre agent actif, destinée à être utilisée dans un procédé de traitement du cancer du sein dans laquelle le taxane est le paclitaxel et l'autre agent actif est la gemcitabine.

2. Combinaison selon la revendication 1 destinée à être utilisée dans un procédé de traitement du cancer du sein, dans laquelle le procédé comprend l'administration au sujet d'une formulation liposomale cationique comprenant 1 mg/m² à 60 mg/m² du taxane; d'une formulation non liposomale comprenant 5 mg/m² à 100 mg/m² du taxane dans la formulation non liposomale; et 100 mg/m² à 1250 mg/m² de l'autre agent actif, et dans laquelle le procédé comprend en particulier l'administration au sujet d'une formulation liposomale cationique comprenant 11 mg/m² à 22 mg/m² du taxane; d'une formulation non liposomale comprenant 70 mg/m² à 90 mg/m² du taxane; et 800 mg/m² à 1250 mg/m² de l'autre agent actif.

3. Combinaison selon la revendication 1 ou 2 destinée à être utilisée dans un procédé de traitement du cancer du sein, dans laquelle la dose totale de taxane dans la formulation liposomale cationique et la formulation non liposomale est entre 80 mg/m² et 120 mg/m².

4. Combinaison selon la revendication 1 ou 2 destinée à être utilisée dans un procédé de traitement du cancer du sein, dans laquelle la formulation liposomale cationique et la formulation non liposomale sont administrées les jours 1, 8 et 15 d'un cycle de traitement de 28 jours, et l'autre agent actif est administré les jours 1 et 8 d'un cycle de traitement de 28 jours, et/ou dans laquelle la formulation liposomale cationique est administrée en premier; la formulation non liposomale est administrée en second; et l'autre agent actif est administré en troisième.

5. Combinaison selon la revendication 1 ou 2 destinée à être utilisée dans un procédé de traitement du cancer du sein, dans laquelle la formulation liposomale cationique est administrée au sujet à un débit de 0,5 mL/min pendant les 15 premières minutes, suivi d'un débit de 1,0 mL/min pendant encore 15 minutes, et suivi d'un débit de 1,5 mL/min après 30 minutes.

6. Combinaison selon la revendication 1 ou 2 destinée à être utilisée dans un procédé de traitement du cancer du sein, dans laquelle la formulation liposomale cationique comprend un lipide cationique de 30 % en mole à 99,9 % en mole et/ou dans laquelle la formulation liposomale cationique comprend un taxane en une quantité d'au moins 0,1 % en mole.

7. Combinaison selon la revendication 1 ou 2 destinée à être utilisée dans un procédé de traitement du cancer du sein, dans laquelle la formulation liposomale cationique comprend un lipide neutre et/ou anionique, dans laquelle la formulation liposomale cationique comprend en particulier un lipide neutre ou anionique en une quantité de 30 % en mole à 55 % en mole, et dans laquelle le lipide neutre peut être le cholestérol, un phospholipide, un lysolipide, un sphingolipide, un lipide pégylé avec une charge neutre, ou un lysophospholipide, en particulier une 1,2-diacyl-sn-glycéro-3-phosphoéthanolamine telle que la 1,2-dioléoyl-sn-glycéro-3-phosphoéthanolamine (DOPE), une 1,2-diacyl-sn-glycéro-3-phosphocholine telle que la 1,2-dioléoyl-sn-glycéro-3-phosphocholine (DOPC) ou une sphingomyéline.

8. Combinaison selon la revendication 1 ou 2 destinée à être utilisée dans un procédé de traitement du cancer du sein, dans laquelle la formulation liposomale cationique a un potentiel zêta positif dans une solution de KCl environ 0,05 M à environ pH 7,5 à température ambiante.

9. Combinaison selon la revendication 1 ou 2 destinée à être utilisée dans un procédé de traitement du cancer du sein, dans laquelle la formulation liposomale cationique comprend un DOTAP, de la DOPC et du paclitaxel, et dans laquelle la formulation liposomale cationique comprend en particulier un DOTAP, de la DOPC et du paclitaxel dans un rapport molaire d'environ 50:47:3.

10. Combinaison selon la revendication 1 ou 2 destinée à être utilisée dans un procédé de traitement du cancer du sein, dans laquelle le statut des récepteurs d'hormones du sujet est négatif à la fois pour le récepteur des œstrogènes et le récepteur de la progestérone, et le cancer du sein est HER2 positif (HER2+), ou dans laquelle le statut des récepteurs d'hormones du sujet est négatif à la fois pour le récepteur des œstrogènes et le récepteur de la progestérone, et le cancer du sein est HER2 négatif (HER2-).

11. Combinaison selon la revendication 1 ou 2 destinée à être utilisée dans un procédé de traitement du cancer du sein, dans laquelle le lipide cationique est un sel de N-[1-(2,3-dioléoyloxy)propyl]-N,N,N-triméthylammonium (DOTAP); le bromure de diméthyldioctadécylammonium (DDAB); un 1,2-diacyloxy-3-triméthylammonium propane; la N-[1-(2,3-dioloyloxy)propyl]-N,N-diméthylamine (DODAP); un 1,2-diacyloxy-3-diméthylammonium propane; le chlorure de N-[1-(2,3-dioléyloxy)propyl]-N,N,N-triméthylammonium (DOTMA); un 1,2-dialkyloxy-3-diméthylammonium propane; la dioctadécylamidoglycyl-spermine (DOGS); le 3β-[N-(N',N'-diméthylamino-éthane)carbamoyl]cholestérol (DC-Chol); le trifluoroacétate de 2,3-dioléoyloxy-N-(2-(sperminecarboxamido)-éthyl)-N,N-diméthyl-1-propanaminium (DOSPA); le β-alanylcholestérol; le bromure de cétyltriméthylammonium (CTAB); la diC14-amidine; la N-tert-butyl-N'-tétradécyl-3-tétradécylamino-propionamidine; 14Dea2; le chlorure de N-(alpha-triméthylammonioacétyl)didodécyl-D-glutamate (TMAG); le chlorure de O,O'-ditétradécanoyl-N-(triméthylammonioacétyl)diéthanolamine; le 1,3-dioléoyloxy-2-(6-carboxyspermyl)-propylamide (DOSPER); l'iodure de N,N,N',N'-tétraméthyl-N,N'-bis(2-hydroxyléthyl)-2,3-dioléoyloxy-1,4-butane-diammonium, un chlorure de 1-[2-(acyloxy)éthyl]2-alkyl(alcényl)-3(2-hydroxyéthyl)-imidazolinium, le bromure de 1,2-dioléoyl-3-diméthyl-hydroxyéthylammonium (DORI); le bromure de 1,2-dioléyloxypropyl-3-diméthylhydroxyéthylammonium (DORIE); le bromure de 1,2-dioléyloxypropyl-3-diméthylhydroxypropylammonium (DORIE-HP); le bromure de 1,2-dioléyloxypropyl-3-diméthylhydroxybutylammonium (DORIE-HS); le bromure de 1,2-dioléyloxypropyl-3-diméthylhydroxypentylammonium (DORIE-Hpe); le bromure de 1,2-dimyristyloxypropyl-3-diméthylhydroxyléthylammonium (DMRIE); le bromure de 1,2-dipalmityloxypropyl-3-diméthylhydroxyéthyl-ammonium (DPRIE); le bromure de 1,2-distéryloxypropyl-3-diméthylhydroxy-éthylammonium (DSRIE); ou une 1,2-diacyl-sn-glycérol-3-éthylphosphocholine, et dans laquelle le chlorure de 1-[2-(acyloxy)éthyl]2-alkyl(alcényl)-3-(2-hydroxyéthyl)-imidazolinium est en particulier le chlorure de 1-[2-(9(Z)-octadécénoyloxy)éthyl]-2-(8(Z)-heptadécényl-3-(2-hydroxyéthyl)-imidazolinium (DOTIM) ou le chlorure de 1-[2-(hexadécanoyloxy)éthyl]-2-pentadécyl-3-(2-hydroxyéthyl)imidazolinium (DPTIM).
